# EUROPEAN PATENT SPECIFICATION

(11) **EP 1 381 356 B1**
(45) Date of publication and mention of the grant of the patent: **28.05.2008**
(21) Application number: 02763962.4
(22) Date of filing: 04.04.2002
(51) Int. Cl.: A61K 31/165, A61P 25/00

(54) **USE OF (Z)-2-CYANO-3-HYDROXY-BUT-2-ENOIC ACID-(4'-TRIFLUOROMETHYLPHENYL)-AMIDE FOR TREATING MULTIPLE SCLEROSIS**
VERWENDUNG VON (Z)-2-CYANO-3-HYDROXY-BUT-2-ENSÄURE-(4'-TRIFLUOROMETHYLPHENYL)-AMID ZUR BEHANDLUNG DER MULTIPLEN SKLEROSE
UTILISATION DE L'ACIDE (Z)-2-CYANO-3-HYDROXY-BUT-2-ENOIQUE-(4'-TRIFLUOROMETHYLPHENYL)-AMIDE POUR LE TRAITEMENT DE LA SCLEROSE EN PLAQUES

(30) Priority: 05.04.2001 US 281685 P; 02.10.2001 GB 0123571
(43) Date of publication of application: 21.01.2004
(60) Divisional application: 08004647.7
(73) Proprietor: Aventis Pharmaceuticals Inc., Bridgewater, NJ 08807 (US)
(72) Inventor: WETTSTEIN, Joseph, Lebanon, NJ 08833 (US)
(74) Representative: Wein, Elmar Michael
(86) International application number: PCT/US2002/010773
(87) International publication number: WO 2002/080897

(56) References cited:
- US-A- 5 780 592
- KAPLAN M.J.: "Leflunomide Aventis Pharma." CURRENT OPINION IN INVESTIGATIONAL DRUGS, (2001) 2/2 (222-230). , XP001085309
- BERTOLINI G. ET AL: "A new rational hypothesis for the pharmacophore of the active metabolite of leflunomide, a potent immunosuppressive drug." JOURNAL OF MEDICINAL CHEMISTRY, (1997) 40/13 (2011-2016). , XP001084038
- MILJKOVIC, D. ET AL: "Leflunomide inhibits activation of inducible nitric oxide synthase in rat astrocytes" BRAIN RESEARCH (2001), 889(1,2), 331-338 , XP001085215

## Description

### FIELD OF THE INVENTION

The present invention relates to methods of treating multiple sclerosis. In particular, the present invention relates to the treatment of multiple sclerosis with (Z)-2-cyano-3-hydroxy-but-2-enoic acid-(4'-trifluoromethylphenyl)-amide, commonly known as teriflunomide.

### BACKROUND OF THE INVENTION

(Z)-2-cyano-3-hydroxy-but-2-enoic acid-(4'-trifluoromethylphenyl)-amide (teriflunomide) has the structure illustrated in Formula 1:

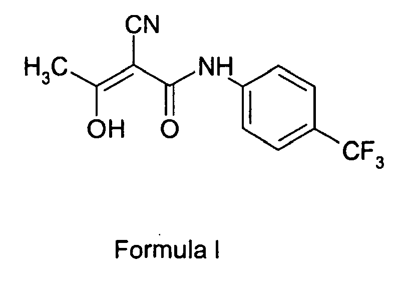

It is an active metabolite of the disease-modifying, antirheumatic drug 5-methylisoxazole-4-carboxylic -(4-trifluoromethyl)-anilide commonly known as leflunomide, the structure of which is shown in Formula II. Leflunomide was first disclosed generically in U.S. Patent 4,087,535, issued on May 2, 1978 and specifically in U.S. Patent 4,284,786, issued on August 18, 1981, wherein it was disclosed that the compound could be used for the treatment of multiple sclerosis.

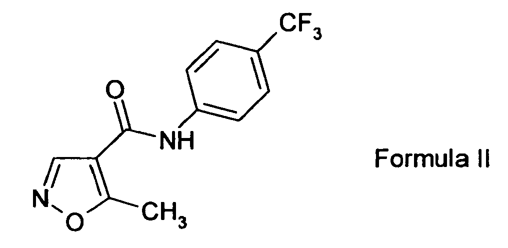

(Z)-2-cyano-3-hydroxy-but-2-enoic acid-(4'-trifluoromethylphenyl)-amide (teriflunomide, Formula I) use in treating chronic graft-versus-host disease has been disclosed in U.S. Patent 4,965,276 issued on October 23, 1990, incorporated herein by reference. U.S. Patent 5,459,163 issued on October 21, 1997 and U.S. Patent 5,679,709 issued on October 21, 1997 disclose compositions useful for treating autoimmune diseases in particular lupus erythematosus. Both of the aforementioned patents are incorporated herein by reference. Teriflunomide has been shown to produce antiproliferative effects on a wide variety of immune cells and cell lines (Cherwinski H. M., et al., J Pharmacol. Exp. Ther. 1995;272:460-8; Prkash A., et al., Drugs 1999;58(6):1137-66; Bartlett R. R. et al., Agent Action 1991;32(1-2):10-21). Additionally, it inhibits the enzyme dihydrooate dehydrogenase, an enzyme essential for the synthesis of pyrimidines (Bruneau J-M, et al., Biochem. J. 1998; 36:299-303). US 5,780,592 discloses the use of compositions comprising lipoproteins and crotonamide derivatives for the treatment of diseases such as an immunological disorder, rejection reactions after organ transplantation or carcinomatous disorder. A new rational hypothesis for the pharmacophore of the "active metabolite" of leflunomide is described in G. Bertolini et al. (J. Med. Chem., 1997, 40, 2011-2016). Leflunomide shows several in vitro effects on iNOS-mediated NO synthesis in several animal cell lines and discusses several hypothesises, which possible mechanisms could be behind leflunomide's effects in animal models and further, in vitro effects of teriflunomide, were described (Dj. Miljkovic et al. (Brain Research (2001), 331-338).

Multiple sclerosis (MS) is a debilitating, inflammatory, neurological illness characterized by demyelination of the central nervous system. The disease primarily affects young adults with a higher incidence in females. Symptoms of the disease include fatigue, numbness, tremor, tingling, dysesthesias, visual disturbances, dizziness, cognitive impairment, urologic dysfunction, decreased mobility, and depression. Four types classify the clinical patterns of the disease: relapsing-remitting, secondary progressive, primary-progressive and progressive-relapsing (S.L. Hauser and D.E. Goodkin, Multiple Sclerosis and Other Demyelinating Diseases in Harrison's Principles of Internal Medicine 14th Edition, vol. 2, Mc Graw-Hill, 1998, pp. 2409-2419).

The exact etiology of MS is unknown; however, it is strongly suspected that the demyelination characteristic of the disease is the result of an autoimmune response perhaps triggered by an environmental insult, e.g. a viral infection. Specifically, it is hypothesized that MS is caused by a T-cell-mediated, autoimmune inflammatory reaction. The autoimmune basis is strongly supported by the fact that antibodies specific to myelin basic protein (MBP) have been found in the serum and cerebrospinal fluid of MS patients and these antibodies along with T-cells that are reactive to MBP and other myelin proteolipids increase with disease activity. Furthermore, at the cellular level it is speculated that T-cell proliferation and other cellular events, such as activation of B cells and macrophages and secretion of cytokines accompanied by a breakdown of the blood-brain barrier can cause destruction of myelin and oligodendrocytes. (R.A. Adams, M.V. Victor and A.H. Ropper eds, Principles of Neurology, Mc Graw-Hill, New York, 1997, pp.903-921). Progressive MS (primary and secondary may be based on a nuerodegenerative process occurring with demyelination.

At the present time there is no cure for MS. Current therapies are aimed at alleviating the symptoms of the disease and arresting its progress, as much as possible. Depending upon the type, drug treatment usually entails the use of disease-modifying agents such as the interferons (interferon beta 1-a, beta 1-b and alpha 2), glatiramer acetate or corticosteroids such as methylprednisolone and prednisone. Also, chemotherapeutic agents such as methotrexate, azathioprine, cladribine cyclophosphamide and cyclosporine have been used. All of the above treatments have side-effect liabilities, little or no effect on fatigue and depression, limited effects on relapse rates and on ability to prevent exacerbation of the disease. Treatment with interferons may also induce the production of neutralizing antibodies, which may ultimately decrease the efficacy of this therapy. Therefore, there still exists a strong need for new drugs, which can be used alone or in combination with other drugs to combat the progression and symptoms of MS.

### SUMMARY OF THE PRESENT INVENTION

The present invention is a use of the compound of formula I 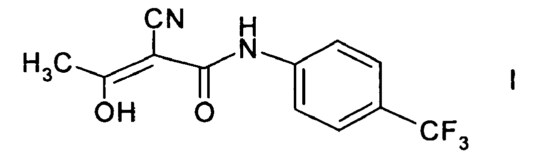 its stereoisomer, or a pharmaceutically acceptable salt thereof, for the manufacture of a medicament for treating multiple sclerosis, wherein said medicament is administered orally.

### DETAILED DESCRIPTION OF THE INVENTION

Terms used herein have the meanings defined in this specification.
a) "Pharmaceutically acceptable salts" means either an acid addition salt or a basic addition salt, whichever is possible to make with the compounds of the present invention.
   "Pharmaceutically acceptable acid addition salt" is any non-toxic organic or inorganic acid addition salt of the base compounds represented by Formula I. Illustrative inorganic acids which form suitable salts include hydrochloric, hydrobromic, sulfuric and phosphoric acid and acid metal salts such as sodium monohydrogen orthophosphate and potassium hydrogen sulfate. Illustrative organic acids which form suitable salts include the mono-, di- and tri-carboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicyclic, 2-phenoxybenzoic, p-toluenesulfonic acid and sulfonic acids such as methanesulfonic acid and 2-hydroxyethanesulfonic acid. Either the mono- or di-acid salts can be formed, and such salts can exist in either a hydrated or substantially anhydrous form. In general, the acid addition salts of these compounds are more soluble in water and various hydrophilic organic solvents and which in comparison to their free base forms, generally demonstrate higher melting points.
   "Pharmaceutically acceptable basic addition salts" means non-toxic organic or inorganic basic addition salts of the compounds of Formula I. Examples are alkali metal or alkaline-earth metal hydroxides such as sodium, potassium, calcium, magnesium or barium hydroxides; ammonia, and aliphatic, alicyclic, or aromatic organic amines such as methylamine, trimethylamine and picoline. The selection of the appropriate salt may be important so that the ester is not hydrolyzed. The selection criteria for the appropriate salt will be known to one skilled in the art.
b) "Patient" means a warm blooded animal, such as for example rat, mice, dogs, cats, guinea pigs, and primates such as humans.
c) "Treat" or "treating" means any treatment, including, but not limited to, alleviating symptoms, eliminating the causation of the symptoms either on a temporary or permanent basis, or preventing or slowing the appearance of symptoms and progression of the named disorder or condition.
d) "Therapeutically effective amount" means an amount of the compound, which is effective in treating the named disorder or condition.
e) "Pharmaceutically acceptable carrier" is a non-toxic solvent, dispersant, excipient, adjuvant or other material which is mixed with the compound of the present invention in order to permit the formation of a pharmaceutical composition, i.e., a dosage form capable of administration to the patient. One example of such a carrier is pharmaceutically acceptable oil typically used for parenteral administration.
f) "Stereoisomers" is a general term for all isomers of the individual molecules that differ only in the orientation of their atoms in space. It includes mirror image isomers (enantiomers), geometric (cis/trans) isomers, and isomers of compounds with more than one chiral center that are not mirror images of one another (diastereoisomers).
g) "Leflunomide" is the generic name for 5-methylisoxazole-4-carboxylic-(4-trifluoromethyl)-anilide.
h) "Teriflunomide " is the generic name for (Z)-2-cyano-3-hydroxy-but-2-enoic acid-(4'-trifluoromethylphenyl)-amide.

The synthesis of the compound of Formula 1 has been disclosed, and is accomplished by methods that are well known to those skilled in the art. For example, US Patent 5,504,084, issued on April 2, 1996, and US Patent 5,990,141, issued on November 23, 1999 disclose methods of synthesis. One synthesis as disclosed in US Patent 5,990,141 is illustrated in Scheme 1.

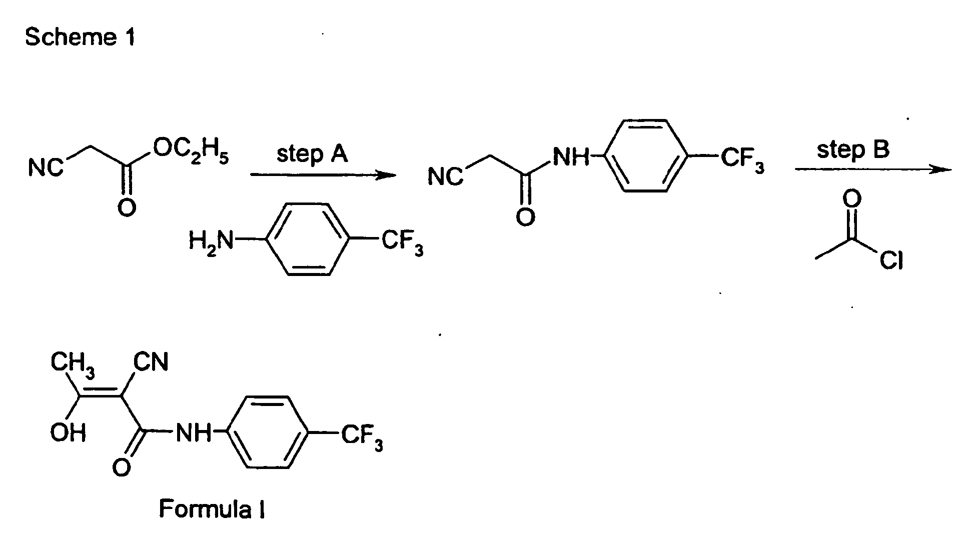

In Scheme 1, step A commercially available cyanoacetic acid ethyl ester is reacted with commercially available 4-trifluoromethylaniline neat at elevated temperature to give cyanoacet-(4-trifluoromethyl)anilide. In step B, the product from step A is dissolved in tetrahydrofuran and reacted with NaH in acetonitrile followed by reaction with acetyl chloride to produce the compound of Formula I.

One method of showing the utility of the present compound as a pharmaceutical that may be useful for the treatment of various conditions associated with MS is its ability to inhibit effects of experimental allergic encephalomyelitis in laboratory animals.

Experimental allergic encephalomyelitis (EAE) is an animal model for MS, which entails inducing a T-cell-mediated autoimmune disease against myelin basic protein in certain susceptible mammalian species. The EAE model is an appropriate method for studying the inflammation of the brain and spinal cord associated with MS (see Bolton, C. Mult. Scler. 1995;1(3);143-9).

In treating a patient afflicted with a condition described above, a compound of Formula (I) is administered orally. One skilled in the art of preparing formulations can determine the proper form and mode of administration depending upon the particular characteristics of the compound selected for the condition or disease to be treated, the stage of the disease, the condition of the patient and other relevant circumstances. For example, see Remington's Pharmaceutical Sciences, 18th Edition, Mack Publishing Co. (1990), incorporated herein by reference.

The compound of the present invention may be administered orally, for example, in the form of tablets, troches, capsules, elixirs, suspensions, solutions, syrups, wafers, chewing gums and the like and may contain one or more of the following adjuvants: binders such as microcrystalline cellulose, gum tragacanth or gelatin; excipients such as starch or lactose, disintegrating agents such as alginic acid, Primogel, corn starch and the like; lubricants such as magnesium stearate or Sterotex; glidants such as colloidal silicon dioxide; and sweetening agents such as sucrose or saccharin may be added or a flavoring agent such as peppermint, methyl salicylate or orange flavoring. When the dosage unit form is a capsule, it may contain, in addition to materials of the above type, a liquid carrier such as polyethylene glycol or a fatty oil. Other dosage unit forms may contain other various materials, which modify the physical form of the dosage unit, for example, as coatings. Thus, tablets or pills may be coated with sugar, shellac, or other enteric coating agents. A syrup may contain, in addition to the present compounds, sucrose as a sweetening agent and certain preservatives, dyes and colorings and flavors.

The dosage range at which the compound of Formula I exhibits its ability to act therapeutically can vary depending upon its severity, the patient, the formulation, other underlying disease states that the patient is suffering from, and other medications that may be concurrently administered to the patient. Generally, the compound of Formula I will exhibit their therapeutic activities at dosages of between about 0.001 mg/kg of patient body weight/day to about 100 mg/kg of patient body weight/day.

### DESCRIPTION OF THE DRAWING

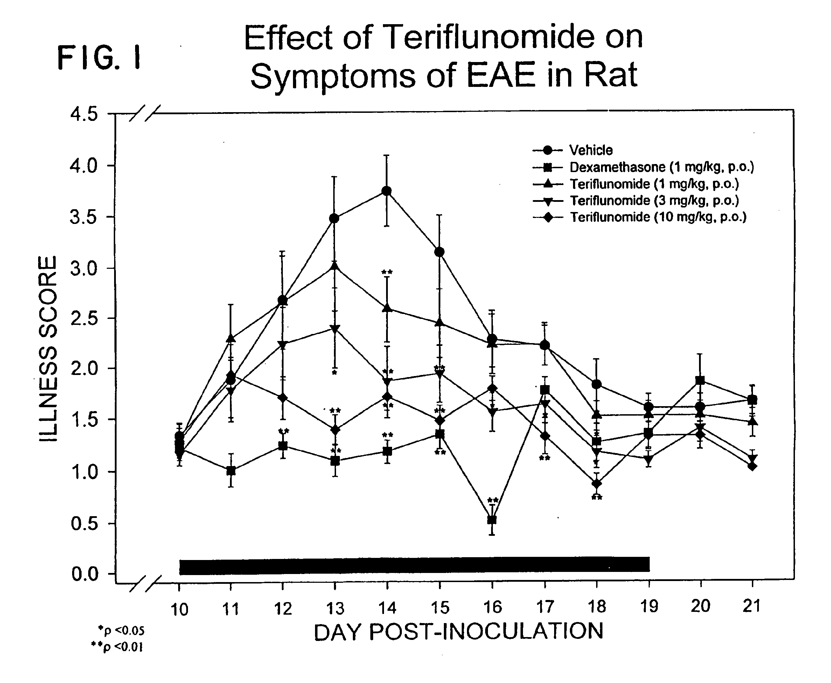

Figure 1 shows the effect of teriflunomide on symptoms in the Rat Experimental Allergic Encephalomyelitis (EAE) at 3 different doses when administered orally (p.o.) as compared to vehicle and dexamethasone.

The following example is being presented to further illustrate the invention.

### Example: Rat Experimental Allergic Encephalomyelitis (Rat EAE)

Experimental allergic encephalomyelitis (EAE) is a T-cell-mediated -autoimmune disease of the nervous system that develops in susceptible animals following sensitization with either whole spinal cord homogenate or a component (myelin basic protein). The EAE rodent model is an appropriate tool for studying the inflammation of the brain and spinal cord observed in MS patients. In rodents, injection of whole spinal cord or spinal cord components such as myelin basic protein induces an autoimmune response based on the activation of T-lymphocytes. Clinical disease typically becomes manifest around day 8-10 after inoculation, observed as a broad spectrum of behavioral anomalies ranging from mild gait disturbances and tail atony to complete paralysis and death. Weight loss typically occurs. In animals that survive, spontaneous recovery occurs, accompanied by variable recovery of most motor function. Depending on the species, allergen, and methodology used, animals tested by the EAE model may experience a single (acute EAE) or several (chronic relapsing EAE) attacks. Several treatment paradigms may be used: the drug or treatment of choice may be administered before immunization, during the nonsymptomatic period or during the clinical disease.

### Animals:

Female Lewis rats, 160-220g (Charles River)

### Antigen:

Whole Guinea Pig spinal cord (Harlan Biosciences).
Complete Freund's adjuvant H37 Ra [1mg/ml Mycobacterium Tuberculosis H37 Ra] (Difco).

### Additional antigen:

Mycobacterium Tuberculosis (Difco).
Bordetella Pertussis [Heat Killed] (Difco).
   Antigen preparation: (for approximately 720 animals)

1. Weigh 5 grams of frozen guinea pig spinal cord.
2. Add 5g spinal cord to 5ml 0.9% saline (1g/ml) in a round bottom centrifuge tube
3. Homogenize on ice with the Tissue-tech until the tissue is completely disrupted (approximately 5 minutes).
4. Add 10 ml Complete Freund's adjuvant H37 Ra supplemented with 200 mg Mycobacterium Tuberculosis (20 mg / ml Complete Freund's adjuvant H37 Ra).
5. Extract homogenate / adjuvant from tube by sucking it into a 10 ml syringe fitted with an 18 gauge emulsifying needle.
6. Emulsify between two 30 ml glass syringes until it becomes difficult to continue passing the material through the needle. (Approximately 5 minutes {there must be no separation between the oil phase and the aqueous phase}).
7. Use immediately or keep on ice until needed (not more than 30 min) (do not freeze).

### Protocol

1. Female Lewis rats (Charles River) are given free access to food and water and should be acclimated a minimum of 3 days before use in experiments.
2. Rats weighing 160 and 220 grams are initially induced with 5% isoflurane (Aerrane, Fort Dodge), 30% O₂, 70% N₂O for 2-5 minutes.
3. The rat is then placed onto a circulating water heating blanket (Gaymar) (dorsal surface up) and into the nose cone for spontaneous respiration of anesthetic gases. The isoflurane is reduced to 2%.
4. Two subcutaneous injections (0.1 ml each) of either antigen or normal saline are made into ventral surface of the hind paws.
5. The animals are removed from the nose cone, weighed and numbered.
6. The rats are allowed to awake from anesthesia and are placed into individual cages.
7. The animals are observed daily for signs of EAE induction (see criteria below)

| | |
|---|---|
| STAGE:0 | NORMAL |
| STAGE 1 | Abnormal gate and tail atony |
| STAGE 2 | Mild but definite weakness of one or both hind legs |
| STAGE: 3 | Severe weakness of one or both hind legs or mild ataxia |
| STAGE: 4 | Severe paraparesis and minimal hind leg movement |
| STAGE: 5 | No hind leg movement and paraplegia |
| STAGE: 6 | Moribund state with no spontaneous movement and impaired respiration. |
| | Increasing degree of front leg involvement and urinary and fecal incontinence may also occur |
| STAGE:7 | DEATH |

Treatment was begun on day 10 after immunization. Since the disease symptoms in this model typically appear 10-11 days after inoculation, this time point may be considered to represent the initial phase of an acute episode of MS. It is judged that this delay of the start of treatment mimics the clinical situation more closely than the traditionally used protocols where drugs are administered at the time of, or even before, inoculation (Teitelbaum D. et al., Proc Natl Acad Sci USA 1999; 96: 3842-3847 and Brod S. A., et al., Ann Neurol 2000; 47: 127-131).

The effect of teriflunomide on symptoms of EAE in rat at various doses is illustrated in Figure 1. Dexamethasone is included in the figure for comparison.

## Claims

1. Use of the compound of formula I 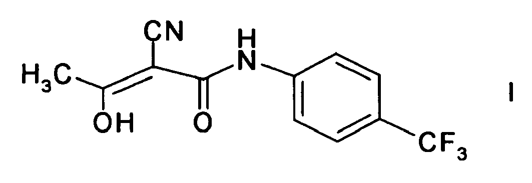 its stereoisomer, or a pharmaceutically acceptable salt thereof, for the manufacture of a medicament for treating multiple sclerosis, wherein said medicament is administered orally.

## Patentansprüche

1. Verwendung der Verbindung der Formel I 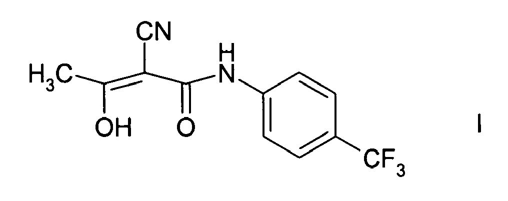 ihres Stereoisomers oder eines pharmazeutisch unbedenklichen Salzes davon zur Herstellung eines Medikaments zur Behandlung von Multipler Sklerose, wobei das Medikament oral verabreicht wird.

## Revendications

1. Utilisation du composé de formule I 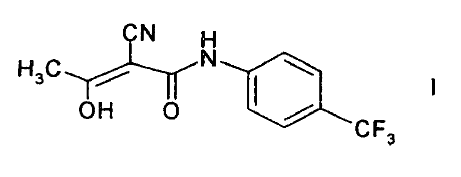 son stéréoisomère, ou un sel pharmaceutiquement acceptable de celui-ci, pour la fabrication d'un médicament destiné au traitement de la sclérose en plaques, ledit médicament étant administré par voie orale.
